(19) Europäisches Patentamt
European Patent Office
Office européen des brevets

(11) Veröffentlichungsnummer: **0 144 052**
**A2**

# (12) EUROPÄISCHE PATENTANMELDUNG

(21) Anmeldenummer: **84114252.4**

(22) Anmeldetag: **26.11.84**

(51) Int. Cl.[4]: **C 07 D 215/20**
A 01 N 47/30, A 01 N 47/38
C 07 D 215/26, C 07 D 215/28

(30) Priorität: **01.12.83 DE 3343416**
**18.05.84 DE 3418532**

(43) Veröffentlichungstag der Anmeldung:
**12.06.85 Patentblatt 85/24**

(84) Benannte Vertragsstaaten:
**BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Hagen, Helmut, Dr.-Ing.**
**Max-Slevogt-Strasse 17e**
**D-6710 Frankenthal(DE)**

(72) Erfinder: **Ziegler, Hans, Dr.-Chem.**
**Am Speyerweg 48**
**D-6704 Mutterstadt(DE)**

(72) Erfinder: **Wuerzer, Bruno, Dr.**
**Ruedigerstrasse 13**
**D-6701 Otterstadt(DE)**

## (54) Chinolinoxyphenylharnstoffe, Verfahren zu ihrer Herstellung und ihre Verwendung zur Bekämpfung unerwünschten Pflanzenwuchhses.

(57) Die Erfindung betrifft Chinolinoxyphenylharnstoffe der Formel

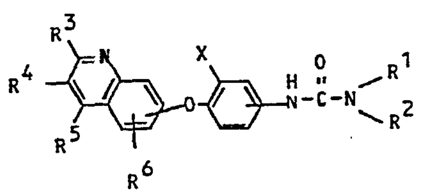

in der

$R^1$ Wasserstoff, Alkyl, Alkoxy oder Aryl,
$R^2$ Wasserstoff, Alkyl oder Alkoxy,
$R^3$, $R^4$ Wasserstoff, Alkyl oder Halogen,
$R^5$ Wasserstoff, Hydroxy, Alkoxy oder Alkyl,
$R^6$ Wasserstoff, Halogen, Nitro oder Alkyl,
X Wasserstoff, Halogen, Alkyl oder Halogenalkyl bedeuten

und die Gruppe $-NR^1R^2$ auch ein 5- oder 6-gliedriger Ring mit gegebenenfalls weiteren Heteroatomen sein kann, Verfahren zu ihrer Herstellung und ihre Verwendung zur Bekämpfung unerwünschten Pflanzenwuchses.

EP 0 144 052 A2

Croydon Printing Company Ltd.

## Chinolinoxyphenylharnstoffe, Verfahren zu ihrer Herstellung und ihre Verwendung zur Bekämpfung unerwünschten Pflanzenwuchses

Die Erfindung betrifft Chinolinoxyphenylharnstoffe, Verfahren zu ihrer Herstellung und Herbizide, die diese Verbindungen als Wirkstoffe enthalten.

Es ist bekannt, daß Naphthoxyphenylharnstoffe herbizid wirksam sind (DE-OS 28 53 791).

Es wurde gefunden, daß Chinolinoxyphenylharnstoffe der Formel

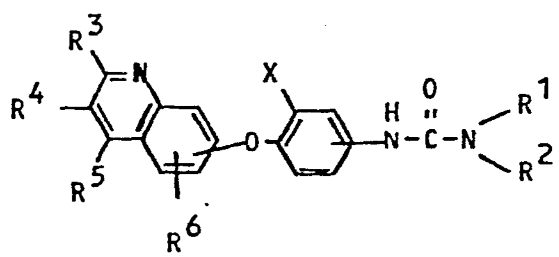

(I),

in der

$R^1$ Wasserstoff, $(C_1-C_4)$-Alkyl, $(C_1-C_4)$-Alkoxy oder Aryl,
$R^2$ Wasserstoff, $(C_1-C_4)$-Alkyl oder $(C_1-C_4)$-Alkoxy,
$R^3$ Wasserstoff, $(C_1-C_4)$-Alkyl oder Halogen,
$R^4$ Wasserstoff, $(C_1-C_4)$-Alkyl oder Halogen,
$R^5$ Wasserstoff, Hydroxy, $(C_1-C_4)$-Alkoxy oder $(C_1-C_4)$-Alkyl,
$R^6$ Wasserstoff, Halogen, Nitro oder $(C_1-C_4)$-Alkyl,
X Wasserstoff, Halogen, $(C_1-C_4)$-Alkyl oder $(C_1-C_4)$-Halogenalkyl

bedeuten und die Gruppe $-NR^1R^2$ auch ein 5- oder 6-gliedriger Ring, der weitere Heteroatome, ausgewählt aus der Gruppe bestehend aus Sauerstoff, Schwefel und Stickstoff, enthalten kann, sein kann, herbizid wirksam und für Kulturpflanzen selektiv sind.

Die als Substituenten $R^1$, $R^2$, $R^3$, $R^4$, $R^5$, $R^6$ und X in Betracht kommenden $(C_1-C_4)$-Alkylreste sind beispielsweise Methyl-, Ethyl-, i-Propyl-, n-Butyl-, vorzugsweise Methylreste. $(C_1-C_4)$-Alkoxyreste für $R^1$, $R^2$ und $R^5$ sind beispielsweise Methoxy-, Ethoxy-, n-Butoxy-, s-Butoxy-, i-Propoxy-, vorzugsweise Methoxyreste. Als Halogenalkylrest kommt für X insbesondere Trifluormethyl in Betracht. Halogensubstituenten in Formel I sind jeweils Chlor, Brom oder Fluor. Aryl für $R^1$ ist insbesondere Phenyl, das durch Halogen, wie Fluor, Chlor, Brom, $(C_1-C_4)$-Alkyl, insbesondere Methyl, Ethyl, $(C_1-C_4)$-Alkoxy, insbesondere Methoxy, oder $(C_1-C_4)$-Halogenalkyl, insbesondere Trifluormethyl, substituiert sein kann.

Die Chinolinoxyphenylharnstoffe der Formel I lassen sich durch Umsetzung von

a) Chinolinoxyanilinen der Formel

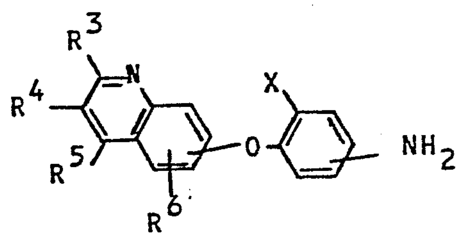

(II),

in der $R^3$, $R^4$, $R^5$, $R^6$ und X die oben genannten Bedeutungen haben,

mit Isocyanaten der Formel

$$O=C=N-R^1 \qquad (III),$$

in der $R^1$ die obengenannte Bedeutung hat, oder

b) durch Umsetzung von Chinolinoxyphenylisocyanaten der Formel

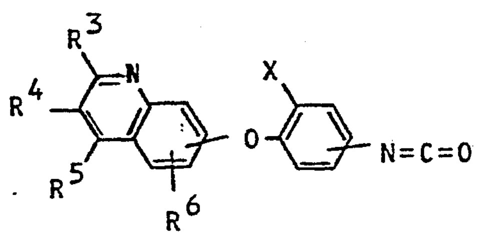

(IV),

in der $R^3$, $R^4$, $R^5$, $R^6$ und X die obengenannten Bedeutungen haben,

mit einem Amin der Formel

$$HNR^1R^2 \qquad (V),$$

in der $R^1$ und $R^2$ die obengenannten Bedeutungen haben, in Gegenwart eines inerten Verdünnungsmittels herstellen.

Für die Umsetzung a) kommen als inerte Lösungsmittel aliphatische oder aromatische Kohlenwasserstoffe, wie Benzin, Benzol oder Toluol, Carbonsäurenitrile, wie Acetonitril, Ketone, wie Aceton, chlorierte Kohlenwasserstoffe, wie Dichlormethan, Chloroform, Tetrachlormethan, acyclische und cyclische Ether, wie Diethylether, Diisopropylether, Tetrahydrofuran und Dioxan, in Betracht. Bevorzugte Lösungsmittel sind Toluol und Dioxan.

Als Katalysator wird eine inerte Base in einem Verhältnis von 1 bis 0,001 Teile, vorzugsweise von 0,02 bis 0,05 Teile, bezogen auf den Ausgangsstoff der Formel II, zugesetzt. Als Base wird Triethylamin bevorzugt.

Ein geeigneter Temperaturbereich für die Umsetzung ist 0 bis 200°C, vorzugsweise wird die Umsetzung zwischen 60 und 100°C durchgeführt.

Für die Umsetzung b) eignen sich die inerten Lösungsmittel, die auch für a) angegeben sind, bevorzugt sind jedoch Acetonitril und Ether, wie Diethylether, Tetrahydrofuran und Dioxan.

Die Umsetzung b) wird bei einer Temperatur im Bereich zwischen -70 und +150°C, vorzugsweise zwischen 0 und 30°C durchgeführt.

Die für die Herstellung der erfindungsgemäßen Verbindungen der Formel I benötigten Chinolinoxyaniline der Formel II können nach Skraup (Org. Reactions 7, 59-98 (1953)) aus Nitrophenoxy-anilinen der Formel

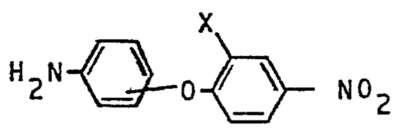
(VI),

in der X die oben genannte Bedeutung hat, oder durch Umsetzung eines Hydroxychinolins der Formel VII mit einem Nitroaromaten der Formel VIII

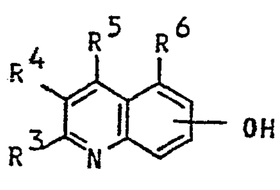
(VII)

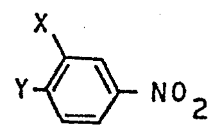
(VIII)

wobei $R^3$, $R^4$, $R^5$, $R^6$ und X die oben genannten Bedeutungen haben und Y Chlor oder Fluor bedeutet (Can.J.Chem. 47, 2015 (1969)), oder durch Umsetzung von einem Chlorchinolin der Formel IX mit einem Nitrophenol der Formel X

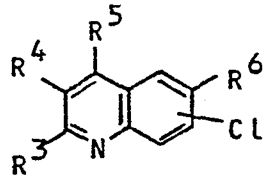
(IX)

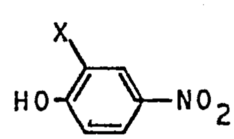
(X)

wobei $R^3$, $R^4$, $R^5$ und X die oben genannten Bedeutungen haben und $R^6$ einen elektronenziehenden Substituenten in o- bzw. p-Position zum Chlor, z.B. eine Cyano- oder Nitrogruppe, bedeutet (Can.J.Chem. 47, 2015 (1969)) und anschließende Reduktion der Nitrogruppe nach einem der üblichen Reduktionsverfahren (Houben-Weyl, Methoden der organ. Chemie, Bd. XI/1, S. 360-515 (1957)) erhalten werden.

Die als Ausgangsprodukte für das Verfahren b) benötigten Isocyanate der Formel IV können aus den Chinolinoxyanilinen der Formel II durch Umsetzung mit Phosgen hergestellt werden (Houben-Weyl, Methoden der organ. Chemie, Bd. VIII, S. 120/121 (1952)).

Die folgenden Beispiele erläutern die Herstellung der Chinolinoxy-nitro--benzole der Formel VI und deren Reduktion zu Chinolinoxyanilinen der Formel II.

Beispiel I

4-(Chinolin-7-oxy)-nitro-benzol

Zu 115 Teilen 3-Phenoxy-4'-nitro-anilin, 297 Teilen Schwefelsäure (98 %ig), 121 Teilen Wasser, 112,5 Teilen 3-Nitrobenzolsulfonsäurenatrium werden bei 120°C 57,5 Teile Glycerin zugegeben. Die Reaktionsmischung wird 4 Stunden auf 140°C erwärmt, auf Eis gegeben, mit Natriumhydroxid neutralisiert und mit Toluol extrahiert. Nach Einengen der Toluolphase und Umkristallisieren aus Toluol/Ligroin erhält man 106 g (entsprechend 80 % d.Th.) 4-(Chinolin-7-oxy)-nitro-benzol vom Schmp. 131°C.

Beispiel II

4-(2-Methyl-chinolin-7-oxy)-nitro-benzol

115 Teile 3-Phenoxy-4'-nitroanilin werden analog Beispiel 1 mit 44 Teilen Crotonaldehyd umgesetzt. Man erhält 105 g (entsprechend 72 % d.Th.) 4-(2--Methyl-chinolin-7-oxy)-nitro-benzol vom Schmp. 105°C.

Beispiel III

4-(3-Methyl-chinolin-7-oxy)-nitro-benzol

115 Teile 3-Phenoxy-4'-nitroanilin werden analog Beispiel 1 mit 44 Teilen Methacrolein umgesetzt. Man erhält 100 g (entsprechend 71 % d.Th.) 4-(3--Methyl-chinolin-7-oxy)-nitro-benzol vom Schmp. 91°C.

Entsprechend werden folgende Chinolinoxy-nitro-benzole der Formel VI erhalten.

Die Reduktion der Verbindungen der Formel VI zu den Chinolinoxyanilinen der Formel II kann nach folgender allgemeiner Vorschrift erfolgen:

Zu einer Mischung von 1009 Teilen $H_2O$, 60 Teilen Eisessig, 1715 Teilen n-Propanol und 605 Teilen Eisenspänen werden bei 90°C dem Molekulargewicht entsprechende Teile eines Nitrophenoxychinolins portionsweise zugegeben. Die Reaktionsmischung wird dann 12 Stunden bei 90°C gerührt und anschließend mit 500 Teilen 20 %iger wäßriger Ammoniaklösung versetzt und heiß filtriert. Das Filtrat wird eingeengt, und die Aminoverbindung wird durch Umkristallisation oder Chromatographie gereinigt. Man erhält das Chinolinoxy-anilin in einer Ausbeute von 60 bis 80 % d.Th.

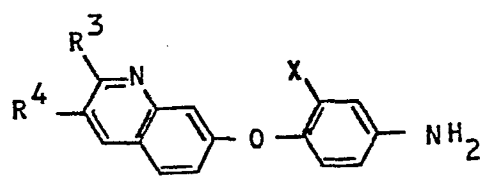

| $R^3$ | $R^4$ | X | Schmp. [°C] |
|---|---|---|---|
| H | H | H | |
| $CH_3$ | H | H | 147 |
| H | $CH_3$ | H | 93 |
| H | H | Cl | 130 |
| $CH_3$ | H | Cl | 136 |
| H | $CH_3$ | Cl | 120 |
| H | H | $CF_3$ | 234 (.HCl) |
| $CH_3$ | H | $CF_3$ | |
| H | $CH_3$ | $CF_3$ | 130 |

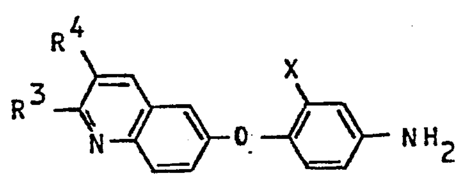

| $R^3$ | $R^4$ | X | Schmp. [°C] |
|---|---|---|---|
| H | H | H | 113 |
| $CH_3$ | H | H | Öl |
| H | $CH_3$ | H | 144 |

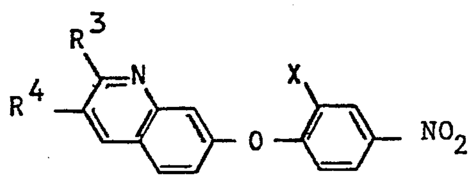

| $R^3$ | $R^4$ | X | Schmp. [°C] |
|---|---|---|---|
| H | H | H | 131 |
| $CH_3$ | H | H | 105 |
| H | $CH_3$ | H | 91 |
| H | H | Cl | Öl |
| $CH_3$ | H | Cl | 170 |
| H | $CH_3$ | Cl | 118 |
| H | H | $CF_3$ | Öl |
| $CH_3$ | H | $CF_3$ | Öl |
| H | $CH_3$ | $CF_3$ | 130 |

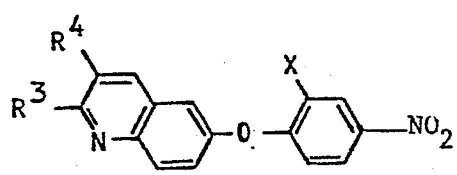

| $R^3$ | $R^4$ | X | Schmp. [°C] |
|---|---|---|---|
| H | H | H | 123 |
| $CH_3$ | H | H | |
| H | $CH_3$ | H | 156 |
| H | H | Cl | 111 |
| $CH_3$ | H | Cl | 150 |
| H | $CH_3$ | Cl | 156 |
| H | H | $CF_3$ | Öl |
| $CH_3$ | H | $CF_3$ | Öl |
| H | $CH_3$ | $CF_3$ | Öl |

| $R^3$ | $R^4$ | X | Schmp. [°C] |
|---|---|---|---|
| H | H | Cl | 107 |
| $CH_3$ | H | Cl | 150 |
| H | $CH_3$ | Cl | |
| H | H | $CF_3$ | 225 (.HCl) |
| $CH_3$ | H | $CF_3$ | Öl |
| H | $CH_3$ | $CF_3$ | Öl |

Die Herstellung der Chinolinoxyphenylharnstoffe der Formel I wird durch folgende Beispiele erläutert:

<u>Beispiel 1</u>

N-[4-(2-Methyl-chinolin-7-oxy)-3-chlor-phenyl]-N'-methyl-harnstoff

28,5 Teile 4-(2-Methyl-chinolin-7-oxy)-3-chlor-anilin werden in 500 Teilen Toluol mit 0,5 Teilen Triethylamin und 10 Teilen Methylisocyanat 12 Stunden bei 80°C gerührt. Der Niederschlag wird abgetrennt und mit Diethylether gewaschen. Man erhält 30,7 g (entsprechend 90 % d.Th.) N-[4-(2-Methyl-chinolin-7-oxy)-3-chlor-phenyl]-N'-methyl-harnstoff vom Schmp. 166°C.

<u>Beispiel 2</u>

N-[4-(2-Methyl-chinolin-7-oxy)-3-chlor-phenyl]-N'-methoxy-N'-methyl-harnstoff

28,5 Teile 4-(2-Methyl-chinolin-7-oxy)-3-chlor-anilin werden bei 0 bis 10°C in eine Lösung von 20 Teilen Phosgen in 600 Teilen Chlorbenzol eingetragen. Die Reaktionsmischung wird 2 Stunden bei 20°C gerührt und anschließend für 45 Minuten auf 90 bis 100°C erwärmt. Danach wird das Lösungsmittel unter vermindertem Druck entfernt.

Der Rückstand wird in 500 Teilen Diethylether suspendiert und mit 10 Teilen Methoxy-methyl-amin versetzt. Die Reaktionsmischung wird 12 Stunden bei Raumtemperatur gerührt, der Niederschlag abgetrennt und mit Wasser und Diethylether behandelt. Man erhält 31 g N-[4-(2-Methyl-chinolin-7-oxy)-3-chlor-phenyl]-N'-methoxy-N'-methyl-harnstoff (entsprechend 83 % d.Th.) vom Schmp. 139°C.

Analog werden folgende Verbindungen der Formel I hergestellt:

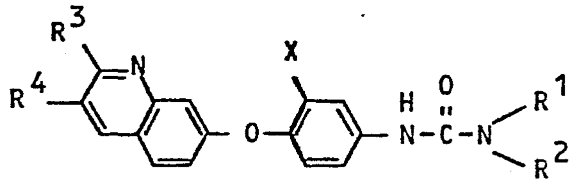

| Bsp. Nr. | $R^1$ | $R^2$ | $R^3$ | $R^4$ | X | Schmp. [°C] |
|---|---|---|---|---|---|---|
| 3 | H | H | H | H | H | |
| 4 | H | $CH_3$ | H | H | H | 151 |
| 5 | $CH_3$ | $CH_3$ | H | H | H | |
| 6 | $CH_3$ | $OCH_3$ | H | H | H | |
| 7 | H | $CH_3$ | $CH_3$ | H | H | 157 |
| 8 | $CH_3$ | $CH_3$ | $CH_3$ | H | H | 166 |
| 9 | $CH_3$ | $OCH_3$ | $CH_3$ | H | H | 99 |
| 10 | H | $CH_3$ | H | $CH_3$ | H | 159 |
| 11 | $CH_3$ | $CH_3$ | H | $CH_3$ | H | 148 |
| 12 | $CH_3$ | $OCH_3$ | H | $CH_3$ | H | Öl |
| 13 | H | H | H | H | Cl | 210 |
| 14 | H | $CH_3$ | H | H | Cl | 62 |
| 15 | $CH_3$ | $CH_3$ | H | H | Cl | 145 |
| 16 | $CH_3$ | $OCH_3$ | H | H | Cl | 117 |
| 17 | $C_2H_5$ | $C_2H_5$ | H | H | Cl | 115 |
| 18 | H | $CH_3$ | $CH_3$ | H | Cl | 166 |
| 19 | $CH_3$ | $CH_3$ | $CH_3$ | H | Cl | 182 |
| 20 | $CH_3$ | $OCH_3$ | $CH_3$ | H | Cl | 159 |
| 21 | H | $CH_3$ | H | $CH_3$ | Cl | - |
| 22 | $CH_3$ | $CH_3$ | H | $CH_3$ | Cl | - |
| 23 | $CH_3$ | $OCH_3$ | H | $CH_3$ | Cl | |
| 24 | H | $CH_3$ | H | H | $CF_3$ | Öl |
| 25 | $CH_3$ | $CH_3$ | H | H | $CF_3$ | Öl |
| 26 | $CH_3$ | $OCH_3$ | H | H | $CF_3$ | Öl |
| 27 | H | $CH_3$ | $CH_3$ | H | $CF_3$ | - |
| 28 | $CH_3$ | $CH_3$ | $CH_3$ | H | $CF_3$ | - |
| 29 | $CH_3$ | $OCH_3$ | $CH_3$ | H | $CF_3$ | - |
| 30 | H | $CH_3$ | H | $CH_3$ | $CF_3$ | - |
| 31 | $CH_3$ | $CH_3$ | H | $CH_3$ | $CF_3$ | 116 |
| 32 | $CH_3$ | $OCH_3$ | H | $CH_3$ | $CF_3$ | - |

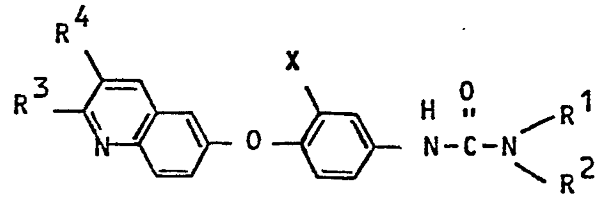

| Bsp. Nr. | $R^1$ | $R^2$ | $R^3$ | $R^4$ | X | Schmp. [°C] |
|---|---|---|---|---|---|---|
| 33 | H | $CH_3$ | H | H | H | - |
| 34 | $CH_3$ | $CH_3$ | H | H | H | 215 |
| 35 | $CH_3$ | $OCH_3$ | H | H | H | 195 |
| 36 | H | $CH_3$ | $CH_3$ | H | H | - |
| 37 | $CH_3$ | $CH_3$ | $CH_3$ | H | H | - |
| 38 | $CH_3$ | $OCH_3$ | $CH_3$ | H | H | - |
| 39 | H | $CH_3$ | H | $CH_3$ | H | 173 |
| 40 | $CH_3$ | $CH_3$ | H | $CH_3$ | H | 173 |
| 41 | $CH_3$ | $OCH_3$ | H | $CH_3$ | H | öl |
| 42 | H | $CH_3$ | H | H | Cl | öl |
| 43 | $CH_3$ | $CH_3$ | H | H | Cl | öl |
| 44 | $CH_3$ | $OCH_3$ | H | H | Cl | öl |
| 45 | H | $CH_3$ | $CH_3$ | H | Cl | 125 |
| 46 | $CH_3$ | $CH_3$ | $CH_3$ | H | Cl | 95 |
| 47 | $CH_3$ | $OCH_3$ | $CH_3$ | H | Cl | öl |
| 48 | H | $CH_3$ | H | $CH_3$ | Cl | 201 |
| 49 | $CH_3$ | $CH_3$ | H | $CH_3$ | Cl | |
| 50 | $CH_3$ | $OCH_3$ | H | $CH_3$ | Cl | |
| 51 | H | $CH_3$ | H | H | $CF_3$ | öl |
| 52 | $CH_3$ | $CH_3$ | H | H | $CF_3$ | öl |
| 53 | $CH_3$ | $OCH_3$ | H | H | $CF_3$ | öl |
| 54 | H | $CH_3$ | $CH_3$ | H | $CF_3$ | |
| 55 | $CH_3$ | $CH_3$ | $CH_3$ | H | $CF_3$ | |
| 56 | $CH_3$ | $OCH_3$ | $CH_3$ | H | $CF_3$ | |
| 57 | H | $CH_3$ | H | $CH_3$ | $CF_3$ | |
| 58 | $CH_3$ | $CH_3$ | H | $CH_3$ | $CF_3$ | |
| 59 | $CH_3$ | $OCH_3$ | H | $CH_3$ | $CF_3$ | |

Die Chinolinoxyphenylharnstoffe der Formel I und ihre Salze können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepukt, wie Kerosin oder Dieselöl, fernr Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, z.B. Methanol, Ethanol, Propanol, Butanol, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden.

Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 8 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung Nr. 11 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N--monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 12 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile des Wirkstoffs Nr. 25 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid

an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile des Wirkstoffs Nr. 51 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile des Wirkstoffs Nr. 46 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile des Wirkstoffs Nr. 53 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 7 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,1 bis 3 kg/ha, vorzugsweise 0,2 bis 1 kg/ha.

Die herbizide Wirkung der Chinolinoxyphenylharnstoffe der Formel I läßt sich durch Gewächshausversuche zeigen:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät.

Zum Zwecke der Nachauflaufbehandlung zieht man die Testpflanzen je nach Wuchsform erst bis zu einer Wuchshöhe von 3 bis 15 cm an und behandelt sie danach. Die Sojapflanzen werden in einem mit Torfmull (peat) angereicherten Substrat angezogen. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Wirkstoffe werden in Wasser als Verteilungsmittel suspendiert oder emulgiert und mittels fein verteilender Düsen gespritzt. Die Aufwandmengen variieren zwischen 0,5 und 3,0 kg Wirkstoff/ha.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

Avena sativa (Hafer), Cassia spp. (Gewürzrinde), Centaurea cyanus (Kornblume), Galium aparine (Klettenlabkraut), Glycine max (Sojabohnen), Ipomoea spp. (Prunkwindearten), Mercurialis annua (Einjähriges Bingelkraut), Zea mays (Mais), Triticum aestivum (Weizen).

Bei einer Aufwandmenge von 3,0 kg Wirkstoff/ha zeigen beispielsweise die Verbindungen Nr. 7, 8, 9, 11, 12, 18, 20, 24, 25, 26, 46, 51, 52 und 53 eine beachtliche herbizide Wirkung. Eine herbizide Wirkung gegen das wichtige breitblättrige Unkraut Cassia spp. und andere bei gleichzeitiger Selektivität in Sojabohnen haben die Verbindungen 8, 12, 15, 16 und 18 bei einer Aufwandmenge von 1,0 bzw. 0,5 kg Wirkstoff/ha. Die Verbindung Nr. 25 bekämpft bei einer Aufwandmenge von 1,0 kg Wirkstoff/ha breitblättrige Unkräuter in Mais. Bei der gleichen Aufwandmenge bekämpfen beispielsweise die Verbindungen Nr. 47, 52 und 53 breitblättrige Unkräuter in Weizen.

In Anbetracht der Verträglichkeit und der Vielzahl der Applikationsmethoden können die erfindungsgemäßen Verbindungen noch in einer weiteren Zahl von Kulturpflanzen zur Beseitigung unerwünschter Wildgräser oder grasartiger Kulturp anzen, sofern sie an gewissen Standorten unerwünscht sind, eingesetzt werden. In Betracht kommen beispielsweise folgende Kulturen:

| Botanischer Name | Deutscher Name |
|---|---|
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. rapa | Weiße Rübe |
| Camellia sinensis | Teestrauch |
| Carthamus tinctorius | Saflor - Färberdistel |
| Caryg illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora,<br>Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glyoine max | Sojabohne |
| Gossypium hirsutum<br>(Gossypium arboreum<br>Gossypium herbaceum<br>Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |

| Botanischer Name | Deutscher Name |
|---|---|
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Theobroma cacao | Kakaobaum |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Chinolinoxyphenylharnstoffe der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe anderer Struktur, Diphenylether, Triazinone, Uracile, Benzofuranderivate, Cyclohexan-1,3-dionderivate und andere herbizide Wirkstoffe in Betracht.

Außerdem kann es von Nutzen sein, die neuen Verbindungen allein oder in Kombination mit anderen Herbiziden, auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Bekämpfung von Ernährungs- und Spurenelementmängeln eingesetzt werden.

0144052

Patentansprüche

1. Chinolinoxyphenylharnstoffe der Formel

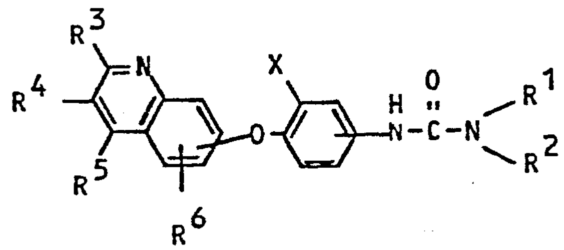

(I),

in der

$R^1$ Wasserstoff, $(C_1-C_4)$-Alkyl, $(C_1-C_4)$-Alkoxy oder Aryl,
$R^2$ Wasserstoff, $(C_1-C_4)$-Alkyl oder $(C_1-C_4)$-Alkoxy,
$R^3$ Wasserstoff, $(C_1-C_4)$-Alkyl oder Halogen,
$R^4$ Wasserstoff, $(C_1-C_4)$-Alkyl oder Halogen,
$R^5$ Wasserstoff, Hydroxy, $(C_1-C_4)$-Alkoxy oder $(C_1-C_4)$-Alkyl,
$R^6$ Wasserstoff, Halogen, Nitro oder $(C_1-C_4)$-Alkyl,
X Wasserstoff, Halogen, $(C_1-C_4)$-Alkyl oder $(C_1-C_4)$-Halogenalkyl

bedeuten und die Gruppe $-NR^1R^2$ auch ein 5- oder 6-gliedriger Ring, der weitere Heteroatome, ausgewählt aus der Gruppe bestehend aus Sauerstoff, Schwefel und Stickstoff, enthalten kann, sein kann.

2. Chinolinoxyphenylharnstoffe der Formel I gemäß Anspruch 1, dadurch gekennzeichnet, daß $R^1$ Wasserstoff oder Methyl und $R^2$ Methyl oder Methoxy bedeuten.

3. Chinolinoxyphenylharnstoffe der Formel I gemäß Anspruch 1, dadurch gekennzeichnet, daß $R^3$ und $R^4$ Wasserstoff oder $(C_1-C_4)$-Alkyl und $R^5$ und $R^6$ Wasserstoff bedeuten.

4. Chinolinoxyphenylharnstoffe der Formel I gemäß Anspruch 1, dadurch gekennzeichnet, daß X Halogen oder $(C_1-C_4)$-Halogenalkyl bedeutet.

5. Verfahren zur Herstellung von Chinolinoxyphenylharnstoffen der Formel I, dadurch gekennzeichnet, daß man

a) Chinolinoxyanilin der Formel

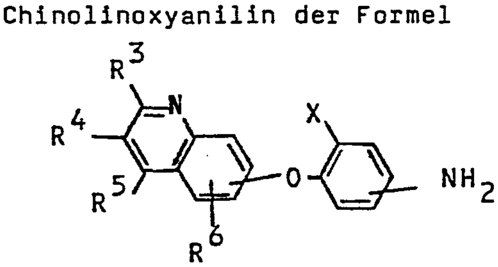

(II),

in der $R^3$, $R^4$, $R^5$, $R^6$ und X die im Anspruch 1 genannten Bedeutungen haben,

mit einem Isocyanat der Formel

$$O{=}C{=}N{-}R^1 \qquad (III),$$

in der $R^1$ die im Anspruch 1 genannten Bedeutungen hat, oder

b) ein Chinolinoxyphenylisocyanat der Formel

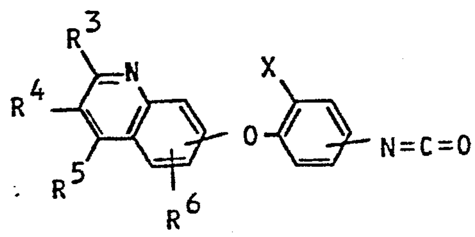

(IV),

in der $R^3$, $R^4$, $R^5$, $R^6$ und X die im Anspruch 1 genannten Bedeutungen haben,

mit einem Amin der Formel

$$HNR^1R^2 \qquad (V),$$

in der $R^1$ und $R^2$ die im Anspruch 1 genannten Bedeutungen haben, in Gegenwart eines inerten Verdünnungsmittels umsetzt.

6. Herbizid, enthaltend einen Chinolinoxyphenylharnstoff der Formel I gemäß Anspruch 1.

7. Herbizid, enthaltend inerte Zusatzstoffe und einen Chinolinoxyphenylharnstoff der Formel I gemäß Anspruch 1.

8. Herbizid, enthaltend einen Chinolinoxyphenylharnstoff der Formel I gemäß Anspruch 2.

9. Herbizid, enthaltend einen Chinolinoxyphenylharnstoff der Formel I gemäß Anspruch 3.

10. Herbizid, enthaltend einen Chinolinoxyphenylharnstoff der Formel I gemäß Anspruch 4.

11. Verfahren zur Bekämpfung unerwünschten Pflanzenwuchses, dadurch gekennzeichnet, daß man die Pflanzen oder den Boden mit einer herbizid wirksamen Menge eines Chinolinoxyphenylharnstoffs der Formel I gemäß Anspruch 1 behandelt.